# EUROPEAN PATENT APPLICATION

(11) **EP 3 093 052 A1**
(43) Date of publication of application: **16.11.2016**
(21) Application number: 16162848.2
(22) Date of filing: 30.03.2016
(51) Int. Cl.: B01D 11/04, A61K 36/06

(54) **NOVEL PURE SUBSTANCE EXTRACTING METHOD**

(30) Priority: 15.05.2015 TW 104115492
(71) Applicant: Sunway Biotech Co., Ltd., Taipei City 114, (TW)
(72) Inventor: Tzu-Ming, Pan, 114 Taipei City (TW)
(74) Representative: Becker Kurig Straus

(57) **Abstract**

The present invention provides a novel pure substance extracting method. Differing from conventional pure substance extracting method utilizing chromatography technique to treat a red mold product with an extracting and purifying process by using a plurality of organic extracting solution, the present invention merely utilizes water and ethanol solution to extract the pure substances of Monascin and Ankaflavin from the red mold product. Based on experimental results, the precipitate of the substances obtained through this novel extracting method is able to contain high extraction ratio of Monascin (∼97.35%) and Ankaflavin (∼95.16%). Moreover, experimental results also prove that the novel pure substance extracting method includes the advantages of high extraction speed, high extraction efficiency, high extraction percentage, and using single organic solution.

## Description

### BACKGROUND OF THE INVENTION

### 1. Field of the Invention

The present invention relates to the technology field of substance extraction and purification, and more particularly to a novel method for extracting pure substance(s) from the secondary metabolites contained by a red yeast product.

### 2. Description of the Prior Art

*Monascus purpureus* NTU 568 is an excellent local *Monascus purpureus* strain, and which is studied and developed by Tzu-Ming PAN, the professor of Institute of Microbiology and Biochemistry of National Taiwan University, and the R&D team thereof. The *Monascus purpureus* NTU 568 includes the characteristics of: growing rapidly, strong starch hydrolysis, high metabolites production. The basic culture medium for *Monascus purpureus* NTU 568 needs includes 2% rice powder, and the best culture temperature is 30°C, the best culture time is 48 hours and the best culture pressure is 1atm.

To verify the viability of *Monascus purpureus* NTU 568, the strain of *Monascus purpureus* NTU 568 is moved from a slant tube to a culture medium of potato dextrose agar (PDA) for carrying out a culturing process. After 15-day culture, it digs and takes out three mycelium with the size of 1 cm³ from the PDA, and then disposes the three mycelium into a culture fluid having 2% rice powder for next-stage culture. Therefore, after 48-hour culture, the *Monascus purpureus* NTU 568 reveals high viability because the culture fluid shows red color. Herein, it needs to further explain that, the storage method for *Monascus purpureus* NTU 568 is to culture the *Monascus purpureus* NTU 568 on a PDA medium disposed in a slant tube under the store temperature of 4°C. Moreover, the *Monascus purpureus* NTU 568 must be treated with one time sub-cultured per 3 months.

In recent years, health foods are developed vigorously, and multi-functional *Monascus pupureus*-fermented products such as red yeast rice and red yeast dioscorea are gradually paid attention. Nowadays, it is well known that the red yeast rice contains following secondary metabolites:
(1) pigment group, consisting of: red pigments (rubropunctamine and monascorubramine), yellow pigments (ankaflavin and monascin), and orange pigments (rubropunctanin and monascorubrin);
(2) cholesterol-lowering substance, such as monacolin K (also called as lovastatin);
(3) blood pressure lowering substance, such as γ-aminobutyric acid (GABA); and
(4) antioxidants, consisting of dimerumic acid and 3-hydoxy-4-methoxy-benzoic acid.

In order to verify the chemical structure of the monascin and the ankaflavin, the early researchers use thin-layer chromatography (TLC) method to extract the monascin and the ankaflavin from a *Monascus* mycelium. The said TLC method consists of following steps:
step (S1a): collecting and washing the *Monascus* mycelium, and then treating the *Monascus* mycelium with a drying process under room temperature;
step (S2a): grinding the dried *Monascus* mycelium to powder;
step (S3a): treating the powdered *Monascus* mycelium with an extracting process through a first eluent of hexane, so as to obtain an extract liquid;
step (S4a): concentrating the extract liquid, and then storing the concentrated extract liquid under a 0 °C environment for 2 days;
step (S5a): filtering the extract liquid for obtaining a filtrate;
step (S6a): preparing a second eluent of hexane and a cellulose column, and then using column chromatography technique to purify the filtrate, so as to obtain a yellow pigment fraction;
step (S7a): providing a (20cm x 20cm) TLC plate made of silica gel and a third eluent containing 25% ether used to separate an ankaflavin fraction and a monascin fraction out of the yellow pigment fraction, and then scraping out the ankaflavin fraction from the TLC plate;
step (S8a): using a fourth eluent containing methylene chloride and ethyl acetate to treat the ankaflavin fraction with an extracting process, so as to obtain an ankaflavin extract;
step (S9a): drying the ankaflavin extract and then using ethanol to crystallize the ankaflavin extract, so as to obtain a yellow prism; wherein one gram of the yellow prism containing74 mg of ankaflavin.

However, the TLC method can only be used to extract micro-scale substance from the *Monascus* mycelium but to obtain macro-scale substance. For example, if it intends to get 1 g of ankaflavin from the *Monascus* mycelium powder, 20∼100 sheets of the TLC plate must be employed in the step (S6a) of the said TLC method.

In view of the fact that the TLC method cannot be used to extract macro-scale substance from the *Monascus* mycelium powder, a micro-scale substance extracting method is therefore provided by literature 1. Literature 1 is proposed by Toshihiro et.al, titled with "Azaphilones, Furanoisophthalides, and Amino Acids from the Extracts of Monascus pilosus-Fermented Rice (Red-Mold Rice) and Their Chemopreventive Effects", and published on JOURNAL OF AGRICULTURAL AND FOOD CHEMISTRY. The said micro-scale substance extracting method consists of following steps:
step (S1b): using 12 L of 70% ethanol to extract red yeast rice (1.5 kg) for 30 minutes;
step (S2b): filtering the product of the step (S2b) so as to obtain a filtrate, and then using 3 L of 70% ethanol to wash the filtrate;
step (S3b): concentrating the product of the step (S2b) under a 50 °C environment;
step (S4b): removing the ethanol in the product of the step (S3b), so as to obtain a first crude extract with 152.9 g;
step (S5b): adding the first crude extract into 1 L of water, and then treating the first crude extract with five times liquid-liquid extraction by using 0.5 L of ethyl acetate as eluent;
step (S6b): concentrating the ethyl acetate extract of the step (S5b) under a 50 °C environment;
step (S7b): removing the ethyl acetate in the product of the step (S6b), so as to obtain a second crude extract with 26.1 g;
step (S8b): preparing silica gel column and eluents, and then using column chromatography technique to separate 11 extract fractions from the second crude extract; wherein the eluents consists of: n-hexane/ethyl acetate = 1/0, n-hexane/ethyl acetate = 9: 1, hexane/ethyl acetate = 4:1, hexane/ethyl acetate = 1:1, hexane/ethyl acetate = 3:7, n-hexane/ethyl acetate = 1:9, hexane/ethyl acetate = 0/1, ethyl acetate/methanol = 9:1, ethyl acetate/methanol = 1:1, and ethyl acetate/methanol = 0/1 (v / v, volume ratio);
step (S9b): taking C fraction out of the 11 extract fractions (A∼K) obtained from the step (S8b) ;
step (S10b): providing an eluent consisting of methanol /water /acetic acid=75:25:3, and then using a high performance liquid chromatography (HPLC) machine to purify 250 mg of C fraction by controlling the flow rate of the eluent at 2.5 mL/min; therefore, 14 mg of monascin and 4 mg of ankaflavin can be obtained by collecting the extract liquid for 27 minutes and 40 minutes, respectively.

From the above-presented steps (S1b)-(S10b), the engineers skilled in the technology filed of pure substance extraction can find that the micro-scale substance extracting method provided by literature 1 cannot performing the macro-scale yellow pigment extraction with high effciency. To further explain that, because the amount of the yellow pigment obtained through the literature 1's method is 18 mg (containing 14 mg of monascin and 4 mg of ankaflavin), it can easily calculate the yield of the yellow pigment is only 7.2 % (18mg/250mg). So that, it is clear that the micro-scale substance extracting method provided by literature 1 may not be the best yellow pigment extracting method for the *Monascus*-fermented products.

Accordingly, a high performance liquid chromatography (HPLC) method is provided by literature 2, wherein the literature 2 is a China invention patent with patent number of CN101255168B. The HPLC method consists of following steps:
step (S1c): using hexane as an eluent to repeatedly extract red yeast rice (10 g) until obtain a colorless extract liquid;
step (S2c): filtering the colorless extract liquid for obtaining a filtrate;
step (S3c): concentrating the filtrate for obtaining a crude extract;
step (S4c): dissolving the crude extract in a methanol, so as to obtain 50 mL of methanol solution;
step (S5c): filtering the methanol solution, and then obtaining a sample solution;
step (S6c): preparing 80% methanol as an eluent, and then using a high performance liquid chromatography (HPLC) machine to purify the sample solution by controlling the flow rate of the eluent at 5 mL/min; therefore, high-purity (96.761%) monascin and high-purity (99.234%) ankaflavin can be obtained by collecting the extract liquid.

From the above-presented steps (S1c)-(S6c), the engineers skilled in the technology filed of pure substance extraction can find that the HPLC method provided by literature 2 still shows following shortcomings and drawbacks:

Because the HPLC machine can only process 1 mL of sample solution per 80 minutes, the HPLC machine must spend 4000 minutes to process 50 mL of sample solution. Moreover, since the HPLC machine can only produce 0.6 mg of monascin per 80 minutes, it is clear that the HPLC method proposed literature 2 is also not the best yellow pigment extracting method for the *Monascus*-fermented products.

Thus, in view of the fact mentioned above, a high speed counter current chromatograph (HSCCC) is provided by literature 3, wherein the literature 3 is a China invention patent with patent number of CN101864191B. The HSCCC method consists of following steps:
step (S1d): dissolving red yeast rice powder in a methanol solution through water bath method under 60 °C;
step (S2d): concentrating the product of the step (S1d), so as to obtain a crude extract;
step (S3d): preparing 800 mg of the crude extract, and then using a stationary phase to dissolving the crude extract for obtaining a sample solution;
step (S4d): preparing an eluent consisting of: petroleum ether/ethyl acetate/ethanol/water = 2.5:7.5:5:5, and then using a HSCCC machine to purify the sample solution by controlling the flow rate of the eluent at 5 mL/min; therefore, a yellow pigment fraction can be obtained by collecting the extract liquid.
step (S5d): dissolving the yellow pigment fraction by using 85% acetone;
step (S6d): concentrating the product of the step (S5d) under a 60 °C environment; and then, after the temperature is cooled down to 15 °C, the monascin and ankaflavin prisms can be obtained.

From the above-presented steps (S1d)-(S6d), the engineers skilled in the technology filed of pure substance extraction can find that the HSCCC method provided by literature 3 still shows following shortcomings and drawbacks:

(1) it is not easy to prepare the eluent consisting of: petroleum ether/ethyl acetate/ethanol/water = 2.5:7.5:5:5. Moreover, the HSCCC method can only produce or obtain 800 mg of yellow pigment from 12 g of red yeast rice powder.

(2) Moreover, for a preparative HSCCC machine, it must spent 133 minutes to finish the entire extracting process for the 48 g (12x4) of red yeast rice powder. Thus, it seems that the HSCCC method does not show great mass production ability for the yellow pigment. On the other hand, the selling price of the preparative HSCCC machine is about NT$ 10 million, and that is extremely expensive.

Thus, because all of the conventional extracting methods for the yellow pigment of the red yeast products have many shortcomings and drawbacks, the inventor of the present application has made great efforts to make inventive research thereon and eventually provided a novel pure substance extracting method.

### SUMMARY OF THE INVENTION

The primary objective of the present invention is to provide a novel pure substance extracting method. Differing from conventional pure substance extracting method utilizing chromatography technique to treat a red mold product with an extracting and purifying process by using a plurality of organic extracting solution, the present invention merely utilizes water and ethanol solution to extract the pure substances of Monascin and Ankaflavin from the red mold product. Based on experimental results, the precipitate of the substances obtained through this novel extracting method is able to contain high extraction ratio of Monascin (∼97.35%) and Ankaflavin (∼95.16%). Moreover, experimental results also prove that the novel pure substance extracting method includes the advantages of high extraction speed, high extraction efficiency, high extraction percentage, and using single organic solution.

Accordingly, in order to achieve the primary objective of the present invention, the inventor of the present invention provides a first embodiment of the novel pure substance extracting method, comprising steps of:
(1) providing the red yeast product with a specific weight; and then, using a first eluent having a first volume to extract the red yeast product under a first temperature, for a first extraction time;
(2) collecting a first extract liquid obtained from the step (1), and then filtering the first extract liquid for obtaining a first filtrate and a first residue of the red yeast product;
(3) using a second eluent having a second volume to extract the first residue under a second temperature, for a second extraction time;
(4) collecting a second extract liquid obtained from the step (3), and then filtering the second extract liquid for obtaining a second filtrate;
(5) mixing the first filtrate and the second filtrate to a mixed filtrate, and then processing the mixed filtrate to a pure substance.

In the first embodiment of the novel pure substance extracting method according to the present invention, the step (5) comprises following detail steps:
(51) mixing the first filtrate and the second filtrate to the mixed filtrate, and then concentrating the mixed filtrate to a thick and dense filtrate;
(52) dissolving the thick and dense filtrate by using a solvent having a third volume, so as to obtain a sample solution;
(53) stirring the sample solution under a second temperature through water bath method, for a first stir time;
(54) adding a water having a fourth volume into the sample solution, and then stirring the sample solution for a second stir time;
(55) treating centrifugation process to the sample solution obtained from the step (54) by 2000 rpm/min, for a first centrifugation time; and
(56) collecting a precipitate, wherein the precipitate is one kind of the secondary metabolites.

In the first embodiment of the novel pure substance extracting method according to the present invention, the specific weight of the red yeast product is 60 g, and the red yeast product is a powdered red yeast rice or a powdered red yeast dioscorea.

In the first embodiment of the novel pure substance extracting method according to the present invention, both the first eluent and the second eluent are 95% ethanol.

In the first embodiment of the novel pure substance extracting method according to the present invention, both the first volume and the second volume are ranged between 250 mL and 320 mL, and the fourth volume being ranged from 8 mL to 12 mL.

In the first embodiment of the novel pure substance extracting method according to the present invention, the first temperature is ranged between 50 °C and 65 °C, and the second temperature being ranged from 55 °C to 75 °C.

In the first embodiment of the novel pure substance extracting method according to the present invention, both the first extraction time and the second extraction time are ranged between 1 hour and 3 hours.

In the first embodiment of the novel pure substance extracting method according to the present invention, the solvent is 95% ethanol, and the third volume of the solvent being ranged between 8 mL and 12 mL.

In the first embodiment of the novel pure substance extracting method according to the present invention, the both the first stir time and the second stir time are ranged between 3 minutes and 7 minutes.

In the first embodiment of the novel pure substance extracting method according to the present invention, 1 gram of the precipitate containing at least 22.5 mg of monascin and at least 16.83 mg of ankaflavin.

In the first embodiment of the novel pure substance extracting method according to the present invention, the precipitate occupies a weight percentage of the sample solution, and the weight percentage is 25%.

In the first embodiment of the novel pure substance extracting method according to the present invention, the extraction ratio of the monascin and the ankaflavin is 97.35% and 95.16%, respectively.

Moreover, in order to achieve the primary objective of the present invention, the inventor of the present invention provides a second embodiment of the novel pure substance extracting method, comprising steps of:
(1') providing the red yeast product with a specific weight; and then using a first eluent having a first volume to extract the red yeast product under a first temperature, for a first extraction time;
(2') collecting a first extract liquid obtained from the step (1'), and then filtering the first extract liquid for obtaining a first filtrate and a first residue of the red yeast product;
(3') using a second eluent having a second volume to extract the first residue under a second temperature, for a second extraction time;
(4') collecting a second extract liquid obtained from the step (3'), and then filtering the second extract liquid for obtaining a second filtrate;
(5') mixing the first filtrate and the second filtrate to a mixed filtrate, and then processing the mixed filtrate to a pure substance.

In the second embodiment of the novel pure substance extracting method according to the present invention, the step (5) comprises following detail steps:
(51') mixing the first filtrate and the second filtrate to the mixed filtrate;
(52') concentrating the mixed filtrate to a thick and dense filtrate having a third volume, and then adding a water having a fourth volume into the thick and dense filtrate, so as to obtain a first sample solution;
(53') treating centrifugation process to the first sample solution obtained from the step (53') by 2000 rpm/min, for a first centrifugation time;
(54') collecting a first precipitate, wherein the first precipitate;
(55') dissolving the first precipitate in a solvent having a fifth volume, so as to obtain a second sample solution;
(56') stirring the second sample solution under a third temperature through water bath method, for a first stir time;
(57') adding a water having sixth volume into the second sample solution, and then stirring the second sample solution for a second stir time;
(58') treating centrifugation process to the second sample solution obtained from the step (57) by 2000 rpm/min, for a second centrifugation time;
(59') collecting a second precipitate wherein the second precipitate is one kind of the secondary metabolites.

In the second embodiment of the novel pure substance extracting method according to the present invention, the specific weight of the red yeast product is at least 20 kg, and the red yeast product being a powdered red yeast rice or a powdered red yeast dioscorea.

In the second embodiment of the novel pure substance extracting method according to the present invention, both the first eluent and the second eluent are 95% ethanol, and both the first volume and the second volume being at least 60 L.

In the second embodiment of the novel pure substance extracting method according to the present invention, both the first temperature and the second temperature are ranged between 50 °C and 65 °C, and the third temperature being ranged from 55 °C to 75 °C.

In the second embodiment of the novel pure substance extracting method according to the present invention, both the first extraction time and the second extraction time are ranged between 1 hour and 3 hours.

In the second embodiment of the novel pure substance extracting method according to the present invention, both the third volume and the fourth volume are at least 4L, and the sixth volume being ranged from 1.5 L to 2.5 L.

In the second embodiment of the novel pure substance extracting method according to the present invention, both the first centrifugation time and the second centrifugation time are range between 8 minutes and 12 minutes.

In the second embodiment of the novel pure substance extracting method according to the present invention, the solvent is 95% ethanol, and the fifth volume being ranged from 1 L to 2 L.

In the second embodiment of the novel pure substance extracting method according to the present invention, the first stir time is ranged between 10 minutes and 20 minutes, and the second stir time being ranged from 3 minutes to 7 minutes.

In the second embodiment of the novel pure substance extracting method according to the present invention, the 1 gram of the second precipitate containing at least 22.5 mg monascin and at least 16.83 mg ankaflavin.

In the second embodiment of the novel pure substance extracting method according to the present invention, the second precipitate occupies a weight percentage of the second sample solution, and the weight percentage is 25%.

In the second embodiment of the novel pure substance extracting method according to the present invention, the extraction ratio of the monascin is at least 98%, and the extraction ratio of the ankaflavin is at least 98%.

### BRIEF DESCRIPTION OF THE DRAWINGS

The invention as well as a preferred mode of use and advantages thereof will be best understood by referring to the following detailed description of an illustrative embodiment in conjunction with the accompanying drawings, wherein:
FIG. 1A and FIG. 1B show flow charts of a first embodiment for a novel pure substance extracting method according to the present invention;
FIG. 2A and FIG. 2B show flow charts of a second embodiment for the novel pure substance extracting method according to the present invention.

### DETAILED DESCRIPTION OF THE PREFERRED EMBODIMENTS

To more clearly describe a novel pure substance extracting method according to the present invention, embodiments of the present invention will be described in detail with reference to the attached drawings hereinafter.

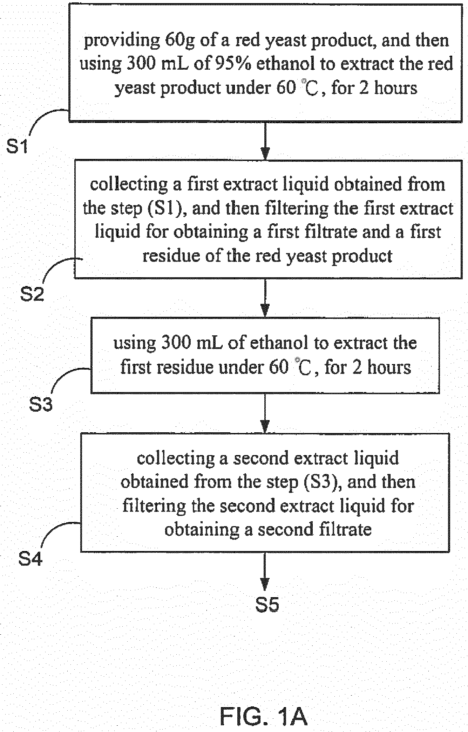

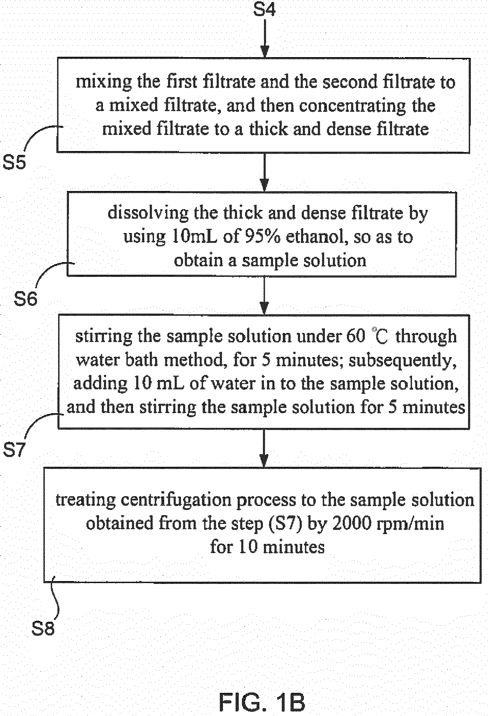

With reference to FIG. 1A and FIG. 1B, where the flow charts of a first embodiment for the novel pure substance extracting method according to the present invention are provided. As shown FIG. 1A and FIG 1B, the first embodiment of the novel pure substance extracting method consisting of following steps:
step (S1): providing 60g of a red yeast product, and then using 300 mL of 95% ethanol to extract the red yeast product under 60 °C, for 2 hours;
step(S2): collecting a first extract liquid obtained from the step (S1), and then filtering the first extract liquid for obtaining a first filtrate and a first residue of the red yeast product;
step(S3): using 300 mL of ethanol to extract the first residue under 60 °C, for 2 hours;
step(S4): collecting a second extract liquid obtained from the step (S3), and then filtering the second extract liquid for obtaining a second filtrate;
step(S5): mixing the first filtrate and the second filtrate to a mixed filtrate, and then concentrating the mixed filtrate to a thick and dense filtrate;
step(S6): dissolving the thick and dense filtrate by using 10mL of 95% ethanol, so as to obtain a sample solution;
step(S7) stirring the sample solution under 60 °C through water bath method, for 5 minutes; subsequently, adding 10 mL of water in to the sample solution, and then stirring the sample solution for 5 minutes;
step(S8): treating centrifugation process to the sample solution obtained from the step (S7) by 2000 rpm/min, for 10 minutes.

After finished the step (S8), the collected precipitate is the one kind secondary metabolites of the red yeast product, i.e., the yellow pigment containing at least 22.5 mg of monascin and at least 16.83 mg of ankaflavin. In the present invention, the red yeast product can be well-known red yeast rice or powdered red yeast dioscorea. Moreover, the present invention does not particularly limit the weight of the said red yeast product must be 60 g, and the volume of 95% ethanol must be 300 mL, the extraction temperature must 60 °C, and the temperature of water bath must be 60 °C. When practically executing the pure substance extracting method proposed by the present invention, the volume of ethanol (i.e., the eluent), the extraction temperature, and the water bath temperature can be ranged respectively from 250 mL to 320 mL, from 50 °C to 65 °C, and from 55 °C and 75 °C.

Moreover, it needs to particularly explain that, the volume of solvent (i.e., the 95% ethanol) applied in step (S6) and the volume of water used in step (S7) must be properly considered and determined. The value of these two volumes can be used to modulate a weight percentage occupied by the precipitate in the sample solution. Moreover, value of these two volumes would also affect the extraction ratio of the yellow pigment of the red yeast product. Following Table 1 records the influence of the volume of solvent applied in step (S6) and the volume of water used in step (S7) on the weight percentage of the precipitate and the extraction ratio of the yellow pigment.

**Table 1**

| | Condition 1 | Condition 2 | Condition 3 | Condition 4 |
|---|---|---|---|---|
| Solvent volume (mL) | 10 | 8 | 7 | 6 |
| Water volume (mL) | 10 | 12 | 13 | 14 |
| Extract ratio of Monascin (%) | 97.99 | **99.8** | 98.51 | 97.35 |
| Extract ratio of Ankaflavin (%) | 98.03 | **99.79** | 97.54 | 95.16 |
| weight percentage of the precipitate (%) | 37.23 | ***43*.*24*** | 25.57 | 27.97 |
| weight percentage of the other impurities (%) | 62.77 | 66.76 | 74.43 | 72.03 |

Therefore, from the Table 1, it can easily find that, the precipitate has a highest weight percentage (43.24%) when the solvent volume and water volume are respectively controlled to be 8 mL and 12 mL. Moreover, the yellow pigments of monascin and ankaflavin also show the highest extract ratio.

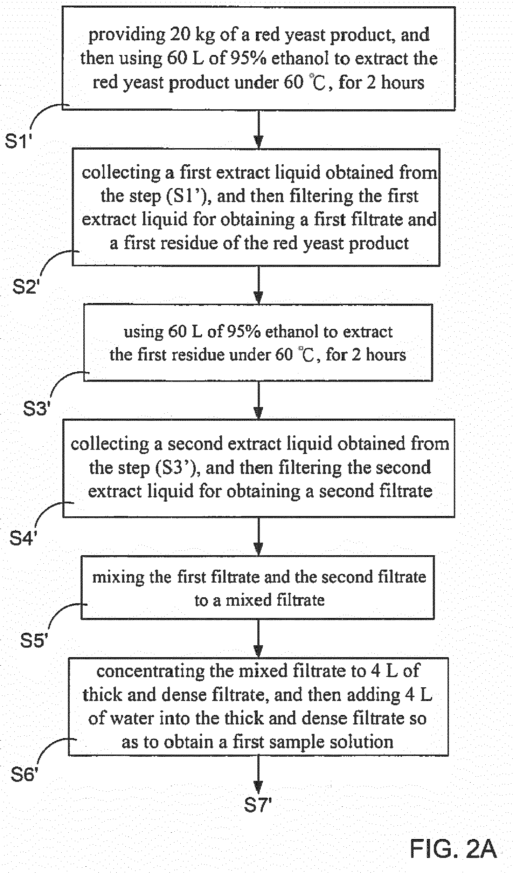

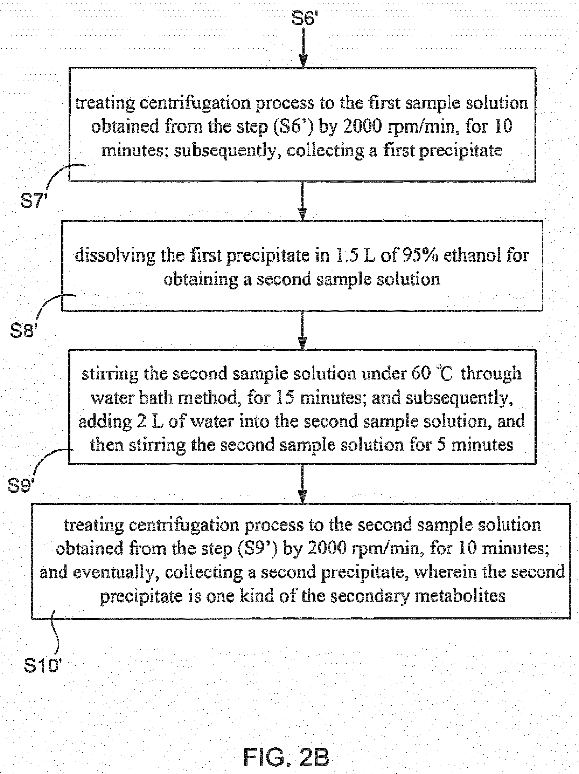

Herein, it is worth noting that the first embodiment of the novel pure substance extracting method is mainly used in laboratory, so as to extract the yellow pigment from the red yeast product with high purity and high extraction ratio. In the invention, the inventors also provide a second embodiment for the novel pure substance extracting method, wherein the second embodiment of the novel pure substance extracting method is primarily applied to mass product the yellow pigment with high purity and high extraction ratio. Please refer to FIG. 2A and FIG. 2B, where the flow charts of the second embodiment for the novel pure substance extracting method according to the present invention are provided. As shown FIG. 2A and FIG. 2B, the first embodiment of the novel pure substance extracting method consisting of following steps:
step (S1'): providing 20 kg of a red yeast product, and then using 60 L of 95% ethanol to extract the red yeast product under 60 °C, for 2 hours;
step(S2'): collecting a first extract liquid obtained from the step (S1'), and then filtering the first extract liquid for obtaining a first filtrate and a first residue of the red yeast product;
step(S3'): using 60 L of 95% ethanol to extract the first residue under 60 °C, for 2 hours;
step(S4'): collecting a second extract liquid obtained from the step (S3'), and then filtering the second extract liquid for obtaining a second filtrate;
step(S5'): mixing the first filtrate and the second filtrate to a mixed filtrate;
step(S6'): concentrating the mixed filtrate to 4 L of thick and dense filtrate, and then adding 4 L of water into the thick and dense filtrate so as to obtain a first sample solution;
step(S7'): treating centrifugation process to the first sample solution obtained from the step (S6') by 2000 rpm/min, for 10 minutes; subsequently, collecting a first precipitate;
step(S8'): dissolving the first precipitate in 1.5 L of 95% ethanol for obtaining a second sample solution;
step(S9'): stirring the second sample solution under 60 °C through water bath method, for 15 minutes; and subsequently, adding 2 L of water into the second sample solution, and then stirring the second sample solution for 5 minutes;
step(S10'): treating centrifugation process to the second sample solution obtained from the step (S9') by 2000 rpm/min, for 10 minutes; and eventually, collecting a second precipitate, wherein the second precipitate is one kind of the secondary metabolites.

In order to prove the collected second precipitate indeed contain the yellow pigments of Monascin and Ankaflavin, following steps are used to separate the monascin and the ankaflavin from the second precipitate:
step(S11'): preparing 662 g of the dried second precipitate and eluents, and then using medium pressure liquid chromatography (MPLC) technique to purity the second precipitate; wherein the eluents consists of: n-hexane/ethyl acetate = 1/0, n-hexane/ethyl acetate = 9:1, n-hexane/ethyl acetate = 8:2, n-hexane/ethyl acetate = 7:3, n-hexane/ethyl acetate = 6:4 (v/v, volume ratio);
step(S12'): taking yellow pigment fraction out of the 5 extract fractions (A-E) obtained from the step (S11');
step (S13'): concentrating the yellow pigment fraction, and then obtain a yellow pigment sample;
step (S14'): dissolving 1 g of the yellow pigment sample in 50 mL of methanol and preparing eluent;
step (S15'): using high performance liquid chromatography (HPLC) machine to purify the yellow pigment sample by controlling the flow rate of the eluent at 15 mL/min, wherein the eluent consists of methanol/water=87:13;
step (S16'): respectively collecting the extract liquid from the HPLC machine for 13.51 minutes and 18.78 minutes, such that 22.5 mg of monascin (purity=99.1%) and 16.83 mg of ankaflavin (purity=99.0%) are obtained.

According to above-presented steps (S1')-(S10') and steps (S11')-(S16'), the person skilled in the pure substance extraction technology filed can easily find that, because the yellow pigment fraction obtained by the step (S13') contains at least 57.5 % of monascin and ankaflavin, it is able to produce 22.5 mg of monascin and 16.83 mg of ankaflavin per 20 minutes by using a preparative HPLC machine.

Therefore, through above descriptions, the novel pure substance extracting method provided by the present invention has been introduced completely and clearly; in summary, the present invention includes the advantages of:
(1) Differing from conventional pure substance extracting method utilizing chromatography technique to treat a red mold product with an extracting and purifying process by using a plurality of organic extracting solution, the present invention merely utilizes water and ethanol solution to extract the pure substances of Monascin and Ankaflavin from the red mold product. So that, the novel pure substance extracting method proposed by the present invention show the advantage of fast extraction speed and without using any organic solvents (except for ethanol).
(2) By using the novel pure substance extracting method proposed by the present invention, it is able to obtain a precipitate containing high-purity monascin and ankaflavin; moreover, the extraction ratio of the monascin and the ankaflavin is up to 97.35% and 95.16%, respectively.
(3) Moreover, differing from conventional HSCCC method, it is able to further produce 22.5 mg of monascin and 16.83 mg of ankaflavin per 20 minutes by using a conventional preparative HPLC machine, without using any expensive preparative HSCCC machine. So that, it is clear that the novel pure substance extracting method reveals the advantages of low cost and great mass production ability on extracting yellow pigments from the red yeast product.

The above description is made on embodiments of the present invention. However, the embodiments are not intended to limit scope of the present invention, and all equivalent implementations or alterations within the spirit of the present invention still fall within the scope of the present invention.

## Claims

1. A novel pure substance extracting method, used for extracting at least one secondary metabolite from a red yeast product and comprising steps of:
(1) providing the red yeast product with a specific weight; and then, using a first eluent having a first volume to extract the red yeast product under a first temperature, for a first extraction time;
(2) collecting a first extract liquid obtained from the step (1), and then filtering the first extract liquid for obtaining a first filtrate and a first residue of the red yeast product;
(3) using a second eluent having a second volume to extract the first residue under a second temperature, for a second extraction time;
(4) collecting a second extract liquid obtained from the step (3), and then filtering the second extract liquid for obtaining a second filtrate;
(5) mixing the first filtrate and the second filtrate to a mixed filtrate, and then processing the mixed filtrate to a pure substance.

2. The novel pure substance extracting method of claim 1, wherein the step (5) comprises following detail steps:
(51) mixing the first filtrate and the second filtrate to the mixed filtrate, and then concentrating the mixed filtrate to a thick and dense filtrate;
(52) dissolving the thick and dense filtrate by using a solvent having a third volume, so as to obtain a sample solution;
(53) stirring the sample solution under a second temperature through water bath method, for a first stir time;
(54) adding a water having a fourth volume into the sample solution, and then stirring the sample solution for a second stir time;
(55) treating centrifugation process to the sample solution obtained from the step (54) by 2000 rpm/min, for a first centrifugation time; and
(56) collecting a precipitate, wherein the precipitate is one kind of the secondary metabolites.

3. The novel pure substance extracting method of claim 1, wherein the specific weight of the red yeast product is 60 g, and the red yeast product being a powdered red yeast rice or a powdered red yeast dioscorea.

4. The novel pure substance extracting method of claim 1, wherein both the first eluent and the second eluent are 95% ethanol.

5. The novel pure substance extracting method of claim 1, wherein both the first volume and the second volume are ranged between 250 mL and 320 mL, and the fourth volume being ranged from 8 mL to 12 mL.

6. The novel pure substance extracting method of claim 1, wherein the first temperature is ranged between 50 °C and 65 °C, and the second temperature being ranged from 55 °C to 75 °C.

7. The novel pure substance extracting method of claim 1, wherein both the first extraction time and the second extraction time are ranged between 1 hour and 3 hours.

8. The novel pure substance extracting method of claim 2, wherein the solvent is 95% ethanol, and the third volume of the solvent being ranged between 8 mL and 12 mL.

9. The novel pure substance extracting method of claim 2, wherein both the first stir time and the second stir time are ranged between 3 minutes and 7 minutes.

10. The novel pure substance extracting method of claim 2, wherein 1 gram of the precipitate containing at least 22.5 mg of monascin and at least 16.83 mg of ankaflavin.

11. The novel pure substance extracting method of claim 2, wherein the precipitate occupies a weight percentage of the sample solution, and the weight percentage is 25%.

12. The novel pure substance extracting method of claim 10, wherein the extraction ratio of the monascin and the ankaflavin is 97.35% and 95.16%, respectively.

13. A novel pure substance extracting method, used for extracting at least one secondary metabolite from a red yeast product and comprising steps of:
(1') providing the red yeast product with a specific weight; and then using a first eluent having a first volume to extract the red yeast product under a first temperature, for a first extraction time;
(2') collecting a first extract liquid obtained from the step (1'), and then filtering the first extract liquid for obtaining a first filtrate and a first residue of the red yeast product;
(3') using a second eluent having a second volume to extract the first residue under a second temperature, for a second extraction time;
(4') collecting a second extract liquid obtained from the step (3'), and then filtering the second extract liquid for obtaining a second filtrate;
(5') mixing the first filtrate and the second filtrate to a mixed filtrate, and then processing the mixed filtrate to a pure substance.

14. The novel pure substance extracting method of claim 13, wherein the step (5') comprises following detail steps:
(51') mixing the first filtrate and the second filtrate to the mixed filtrate;
(52') concentrating the mixed filtrate to a thick and dense filtrate having a third volume, and then adding a water having a fourth volume into the thick and dense filtrate, so as to obtain a first sample solution;
(53') treating centrifugation process to the first sample solution obtained from the step (53') by 2000 rpm/min, for a first centrifugation time;
(54') collecting a first precipitate;
(55') dissolving the first precipitate in a solvent having a fifth volume, so as to obtain a second sample solution;
(56') stirring the second sample solution under a third temperature through water bath method, for a first stir time;
(57') adding a water having sixth volume into the second sample solution, and then stirring the second sample solution for a second stir time;
(58') treating centrifugation process to the second sample solution obtained from the step (57) by 2000 rpm/min, for a second centrifugation time;
(59') collecting a second precipitate wherein the second precipitate is one kind of the secondary metabolites.

15. The novel pure substance extracting method of claim 13, wherein the specific weight of the red yeast product is at least 20 kg, and the red yeast product being a powdered red yeast rice or a powdered red yeast dioscorea.

16. The novel pure substance extracting method of claim 13, wherein both the first eluent and the second eluent are 95% ethanol, and both the first volume and the second volume being at least 60 L.

17. The novel pure substance extracting method of claim 13, wherein both the first temperature and the second temperature are ranged between 50 °C and 65 °C, and the third temperature being ranged from 55 °C to 75 °C.

18. The novel pure substance extracting method of claim 13, wherein both the first extraction time and the second extraction time are ranged between 1 hour and 3 hours.

19. The novel pure substance extracting method of claim 14, wherein both the third volume and the fourth volume are at least 4L, and the sixth volume being ranged from 1.5 L to 2.5 L.

20. The novel pure substance extracting method of claim 14, wherein both the first centrifugation time and the second centrifugation time are range between 8 minutes and 12 minutes.

21. The novel pure substance extracting method of claim 14, wherein the solvent is 95% ethanol, and the fifth volume being ranged from 1 L to 2 L.

22. The novel pure substance extracting method of claim 14, wherein the first stir time is ranged between 10 minutes and 20 minutes, and the second stir time being ranged from 3 minutes to 7 minutes.

23. The novel pure substance extracting method of claim 14, wherein 1 gram of the second precipitate containing at least 22.5 mg monascin and at least 16.83 mg ankaflavin.

24. The novel pure substance extracting method of claim 14, wherein the second precipitate occupies a weight percentage of the second sample solution, and the weight percentage is 25%.

25. The novel pure substance extracting method of claim 24, wherein the extraction ratio of the monascin is at least 98%, and the extraction ratio of the ankaflavin is at least 98%.
